(19) Europäisches Patentamt
European Patent Office
Office européen des brevets

(11) EP 2 883 862 A1

(12) **EUROPÄISCHE PATENTANMELDUNG**

(43) Veröffentlichungstag:
**17.06.2015 Patentblatt 2015/25**

(51) Int Cl.:
***C07C 209/16*** *(2006.01)*

(21) Anmeldenummer: **13196365.4**

(22) Anmeldetag: **10.12.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **BASF SE**
**67056 Ludwigshafen (DE)**

(72) Erfinder:
- **Wigbers, Christof Wilhelm**
  **68167 Mannheim (DE)**
- **Melder, Johann-Peter**
  **67459 Böhl-Iggelheim (DE)**
- **Mägerlein, Wolfgang**
  **67117 Limburgerhof (DE)**
- **Krug, Thomas**
  **67550 Worms (DE)**

(54) **Verfahren zur Herstellung von Aminen**

(57) Verfahren zur Herstellung von Aminen durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines Katalysators, dadurch gekennzeichnet, dass die Querschnittsbelastung im Reaktor im Bereich von 5 kg/(m2 s) bis 50 kg/(m2 s) liegt,

EP 2 883 862 A1

## Beschreibung

Die vorliegende Erfindung betrifft ein kontinuierliches Verfahren zur Herstellung von Aminen durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines Katalysators.

Ein weiterer Gegenstand der vorliegenden Erfindung ist ein Verfahren zur Herstellung von Folgeprodukten aus erfindungsgemäß hergestellten Aminen.

Die Verfahrensprodukte finden u.a. Verwendung als Zwischenprodukte bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

Bei der Umsetzung eines Alkohols, Aldehyds und/oder Ketons mit Wasserstoff und einer Stickstoffverbindung zu den entsprechenden Aminen ist es häufig erforderlich einen hohen Umsetzungsgrad bezüglich des eingesetzten Alkohols, Aldehyds und/oder Ketons zu erzielen, da nicht umgesetzte oder nur teilweise umgesetzte Alkohole, Aldehyde und/oder Ketone nur schwer abzutrennen sind, Nebenreaktionen eingehen können und in den Folgeanwendungen zu unerwünschten Eigenschaften, wie Geruch und Verfärbung, führen können. Es ist weiterhin häufig wünschenswert eine hohe Selektivität bezüglich der Bildung von primären Aminen aus primären Alkoholen zu erzielen und dabei die Bildung von sekundären und tertiären Aminen zu vermeiden.

Verfahren zur Herstellung von Aminen aus Alkoholen mit einem Aminierungsmittel (Alkoholaminierung) bzw. zur Umsetzung von Aldehyden bzw. Ketonen mit einem Aminierungsmittel (reduktive Aminierung) sind beispielsweise im Ullmann (Aliphatic Amines in Ullmann's Encyclopedia of Industrial Chemistry, Karsten Eller, Erhard Henkes, Roland Rossbacher and Hartmut Höke, Published Online : 15 JUN 2000, DOI: 10.1002/14356007.a02_001) beschrieben.

Die Aufgabe der vorliegenden Erfindung bestand darin ein Festbettverfahren für die Herstellung von Aminen durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines Katalysators zur Verfügung zu stellen, das den Einsatz von Katalysatoren bei milderen Reaktionsbedingungen, d.h. insbesondere geringeren Drücken und/oder Temperaturen, ermöglicht.

Ein weiteres Ziel der vorliegenden Erfindung bestand in der Bereitstellung eines Festbettverfahrens, bei dem hohe Ausbeuten und Selektivitäten bei der Aminherstellung erzielt werden können und das zudem wirtschaftlich zu realisieren ist.

Insbesondere sollte bei einem Einsatz von primären Alkoholen und/oder Aldehyden als Ausgangsstoffen die Bildung von sekundären und tertiären Aminen verringert werden bzw. die Verhältnis von primären zu sekundären Aminen eingestellt werden können. Insbesondere bei der Herstellung von Aminoethoxyethanol (ADG) sollte das Verhältnis von ADG zu Morpholin erhöht werden.

Ein weiteres Ziel der vorliegenden Erfindung bestand in der Absenkung des Reaktionsdrucks gegenüber konventionellen Verfahren. Eine Absenkung des Reaktionsdrucks ermöglicht in der Regel eine Verringerung der Investitionskosten, da die Anforderungen bezüglich der Reaktorsicherheit für Niederdruckverfahren nicht so hoch sind, wie für Hochdruckverfahren.

Erfindungsgemäß wurde die Aufgabe durch ein kontinuierliches Verfahren zur Herstellung von Aminen durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines Katalysators, dadurch gekennzeichnet, dass die Querschnittsbelastung im Reaktor im Bereich von 5 kg/($m^2$ s) bis 50 kg/($m^2$ s) liegt, gelöst.

In dem erfindungsgemäßen Verfahren werden primären oder sekundären Alkohole, Aldehyde und/oder Ketone mit Wasserstoff und einer Stickstoffverbindung, ausgewählt aus der Gruppe Ammoniak, primäre und sekundäre Amine, in Gegenwart eines Katalysators zu Aminen umgesetzt.

Nachfolgend wird die Umsetzung von primären oder sekundären Alkoholen, Aldehyde und/oder Ketone mit Wasserstoff und einer Stickstoffverbindung, ausgewählt aus der Gruppe Ammoniak, primäre und sekundäre Amine, als Alkoholaminierung bezeichnet. Die Umsetzung von Aldehyden und/oder Ketonen mit Wasserstoff und einer Stickstoffverbindung, ausgewählt aus der Gruppe Ammoniak, primäre und sekundäre Amine, wird nachfolgend als reduktive Aminierung bezeichnet. Beide Reaktionstypen, also die Alkoholaminierung und die reduktive Aminierung, werden zusammen als Aminierung oder aminierende Hydrierung bezeichnet

Mit dem erfindungsgemäßen Verfahren herstellbar sind z.B. Amine der Formel I

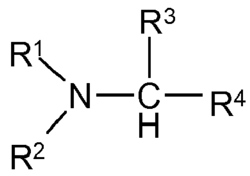

(I),

in der

| | |
|---|---|
| $R^1$, $R^2$ | Wasserstoff (H), Alkyl, wie $C_{1-20}$-Alkyl, Cycloalkyl, wie $C_{3-12}$-Cycloalkyl, Alkoxy-alkyl, wie $C_{2-30}$-Alkoxyalkyl, Dialkylaminoalkyl, wie $C_{3-30}$-Dialkylaminoalkyl, Aryl, Aralkyl, wie $C_{7-20}$-Aralkyl, und Alkylaryl, wie $C_{7-20}$-Alkylaryl, oder gemeinsam $-(CH_2)_j-X-(CH_2)_k-$, |
| $R^3$, $R^4$ | Wasserstoff (H), Alkyl, wie $C_{1-20}$-Alkyl, Cycloalkyl, wie $C_{3-12}$-Cycloalkyl, Hydroxyalkyl, wie $C_{1-20}$-Hydroxyalkyl, Aminoalkyl, wie $C_{1-20}$-Aminoalkyl, Hydroxyalkylaminoalkyl, wie $C_{2-20}$- Hydroxyalkylaminoalkyl, Alkoxyalkyl, wie $C_{2-30}$-Alkoxyalkyl, Dialkylaminoalkyl, wie $C_{3-30}$-Dialkylaminoalkyl, Alkylaminoalkyl, wie $C_{2-30}$-Alkylaminoalkyl, $R^5-(OCR^6R^7CR^8R^9)_n-(OCR^6R^7)$, Aryl, Heteroaryl, Aralkyl, wie $C_{7-20}$-Aralkyl, Heteroarylalkyl, wie $C_{4-20}$-Heteroarylalkyl, Alkylaryl, wie $C_{7-20}$-Alkylaryl, Alkylheteroaryl, wie $C_{4-20}$-Alkylheteroaryl, und $Y-(CH_2)_m-NR^5-(CH_2)_q$ oder gemeinsam $-(CH_2)_l-X-(CH_2)_m-$ oder |
| $R^2$ und $R^4$ | gemeinsam $-(CH_2)_l-X-(CH_2)_m-$, |
| $R^5$, $R^{10}$ | Wasserstoff (H), Alkyl, wie $C_{1-4}$-Alkyl, Alkylphenyl, wie $C_{7-40}$-Alkylphenyl, |
| $R^6$, $R^7$, $R^8$, $R^9$ | Wasserstoff (H), Methyl oder Ethyl, |
| X | $CH_2$, $CHR^5$, Sauerstoff (O), Schwefel (S) oder $NR^5$, |
| Y | $N(R^{10})_2$, Hydroxy, $C_{2-20}$-Alkylaminoalkyl oder $C_{3-20}$-Dialkylaminoalkyl, |
| n | eine ganze Zahl von 1 bis 30 und |
| j, k, l, m, q | eine ganze Zahl von 1 bis 4, |

bedeuten.

Das erfindungsgemäße Verfahren findet daher bevorzugt zur Herstellung eines Amins I Anwendung, indem man einen primären oder sekundären Alkohol der Formel II

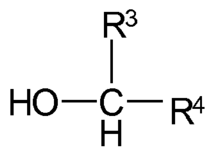

(II)

und/oder Aldehyd und/oder Keton der Formel VI bzw. VII

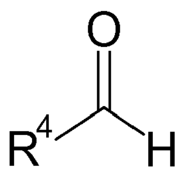

(VI)

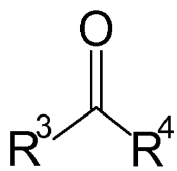

(VII)

mit einer Stickstoffverbindung der Formel III

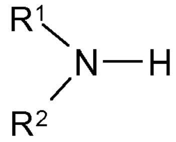

(III),

wobei $R^1$, $R^2$, $R^3$ und $R^4$ die oben genannten Bedeutungen haben, umsetzt.

Bei dem Eduktalkohol kann es sich auch um einen Aminoalkohol handeln, z.B. einem Aminoalkohol gemäß der Formel II.

Wie aus den Definitionen für die Reste $R^2$ und $R^4$ hervorgeht, kann die Umsetzung auch intramolekular in einem entsprechenden Aminoalkohol, Aminoketon oder Aminoaldehyd erfolgen.

Zur Herstellung des Amins I wird demnach rein formal ein Wasserstoffatom der Stickstoffverbindung III durch den Rest $R^4(R^3)CH$- unter Freisetzung von einem Moläquivalent Wasser ersetzt.

Das erfindungsgemäße Verfahren findet auch bevorzugt Anwendung bei der Herstellung eines zyklischen Amins der Formel IV

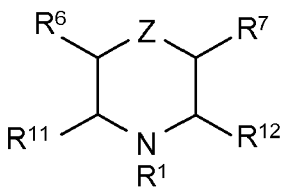

(IV),

in der

| | |
|---|---|
| $R^{11}$ und $R^{12}$ | Wasserstoff (H), Alkyl, wie $C_1$- bis $C_{20}$-Alkyl, Cycloalkyl, wie $C_3$- bis $C_{12}$-Cycloalkyl, Aryl, Heteroaryl, Aralkyl, wie $C_7$- bis $C_{20}$-Aralkyl, und Alkylaryl, wie $C_7$- bis $C_{20}$-Alkylaryl, |
| Z | $CH_2$, $CHR^5$, Sauerstoff (O), $NR^5$ oder $NCH_2CH_2OH$ bedeuten und |
| $R^1$, $R^6$, $R^7$ | die oben genannten Bedeutungen haben, |

durch Umsetzung eines Alkohols der Formel V

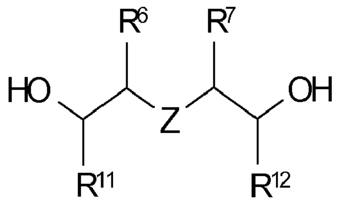

(V)

mit Ammoniak oder einem primären Amin der Formel VIII

**$R^1$- $NH_2$** **(VIII).**

Die Substituenten $R^1$ bis $R^{12}$, die Variablen X, Y, Z und die Indizes j, k, l, m, n und q in den Verbindungen I, II, III, IV, V, VI und VII haben unabhängig voneinander folgende Bedeutungen:

$R^1$, $R^2$, $R^3$, $R^4$, $R^5$, R6, R7, $R^8$, $R^9$, $R^{10}$, $R^{11}$, $R^{12}$:

- Wasserstoff (H),

$R^3$, $R^4$:

- Alkyl, wie $C_{1-20}$-Alkyl, bevorzugt $C_{1-14}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl,

- Hydroxyalkyl, wie $C_{1-20}$-Hydroxyalkyl, bevorzugt $C_{1-8}$-Hydroxyalkyl, besonders bevorzugt $C_{1-4}$-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)-ethyl,

- Aminoalkyl, wie $C_{1-20}$-Aminoalkyl, bevorzugt $C_{1-8}$-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Amino-ethyl)aminomethyl,

- Hydroxyalkylaminoalkyl, wie $C_{2-20}$-Hydroxyalkylaminoalkyl, bevorzugt $C_{3-8}$-Hydroxy-alkylaminoalkyl, wie (2-Hydroxyethylamino)methyl, 2-(2-Hydroxyethylamino)ethyl und 3-(2-Hydroxyethylamino)propyl,

- $R^5$-$(OCR^6R^7CR^8R^9)_n$-$(OCR^6R^7)$, bevorzugt $R^5$-$(OCHR^7CHR^9)_n$-$(OCR^6R^7)$, besonders bevorzugt $R^5$-$(OCH_2CHR^9)_n$-$(OCR^6R^7)$,

- Alkylaminoalkyl, wie $C_{2-30}$-Alkylaminoalkyl, bevorzugt $C_{2-20}$-Alkylaminoalkyl, besonders bevorzugt $C_{2-8}$-Alkylaminoalkyl, wie Methylaminomethyl, 2-Methylaminoethyl, Ethylaminomethyl, 2-Ethylaminoethyl und 2-(iso-Propylamino)ethyl, $(R^5)HN$-$(CH_2)_q$,

- Y-$(CH_2)_m$-$NR^5$-$(CH_2)_q$,

- Heteroarylalkyl, wie $C_{4-20}$-Heteroarylalkyl, wie Pyrid-2-yl-methyl, Furan-2-yl-methyl, Pyrrol-3-yl-methyl und Imidazol-2-yl-methyl,

- Alkylheteroaryl, wie $C_{4-20}$-Alkylheteroaryl, wie 2-Methyl-3-pyridinyl, 4,5-Dimethylimidazol-2-yl, 3-Methyl-2-furanyl und 5-Methyl-2-pyrazinyl,

- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

$R^1$, $R^2$, R3, $R^4$:

- Cycloalkyl, wie $C_{3-12}$-Cycloalkyl, bevorzugt $C_{3-8}$-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,

- Alkoxyalkyl, wie $C_{2-30}$-Alkoxyalkyl, bevorzugt $C_{2-20}$-Alkoxyalkyl, besonders bevorzugt $C_{28}$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl, besonders bevorzugt $C_{2-4}$-Alkoxyalkyl,

- Dialkylaminoalkyl, wie $C_{3-30}$-Dialkylaminoalkyl, bevorzugt $C_{3-20}$-Dialkylaminoalkyl, besonders bevorzugt $C_{3-10}$-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-iso-propylamino)-ethyl, 3-(N,N-Dimethylamino)propyl, $(R^5)_2N$-$(CH_2)_q$,

- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- Alkylaryl, wie $C_{7-20}$-Alkylaryl, bevorzugt $C_{7-12}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

- Aralkyl, wie $C_{7-20}$-Aralkyl, bevorzugt $C_{7-12}$-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

- $R^3$ und $R^4$ oder $R^2$ und $R^4$ gemeinsam eine $-(CH_2)_l$-X-$(CH_2)_m$- Gruppe, wie $-(CH_2)_3-$,$-(CH_2)_4-$, $-(CH_2)_5-$, $-(CH_2)_6-$, $-(CH_2)_7-$, $-(CH_2)$-O-$(CH_2)_2-$, $-(CH_2)$-$NR^5$-$(CH_2)_2-$, $-(CH_2)$-$CHR^5$-$(CH_2)_2-$, $-(CH_2)_2$-O-$(CH_2)_2-$,

$-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^1$, $R^2$:

- Alkyl, wie $C_{1-20}$-Alkyl, bevorzugt $C_{1-8}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt $C_{1-4}$-Alkyl, oder

- $R^1$ und $R^2$ gemeinsam eine $-(CH_2)_j-X-(CH_2)_k-$ Gruppe, wie $-(CH_2)_3-$, $-(CH_2)_4-$, $-(CH_2)_5-$, $-(CH_2)_6-$, $-(CH_2)_7-$, $-(CH_2)-O-(CH_2)_2-$, $-(CH_2)-NR^5-(CH_2)_2-$, $-(CH_2)-CHR^5-(CH_2)_2-$, $-(CH_2)_2-O-(CH_2)_2-$, $-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^5$, $R^{10}$:

- Alkyl, bevorzugt $C_{1-4}$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

- Alkylphenyl, bevorzugt $C_{7-40}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl, insbesondere $C_{7-20}$-Alkylphenyl,

$R^6$, $R^7$, $R^8$, $R^9$:

- Methyl oder Ethyl, bevorzugt Methyl,

$R^{11}$, $R^{12}$:

- Alkyl, wie $C_1$- bis $C_{20}$-Alkyl, Cycloalkyl, wie $C_3$- bis $C_{12}$-Cycloalkyl, Aryl, Heteroaryl, Aralkyl, wie $C_7$- bis $C_{20}$-Aralkyl, und Alkylaryl, wie $C_7$- bis $C_{20}$-Alkylaryl, jeweils wie oben definiert,

X:

- $CH_2$, $CHR^5$, Sauerstoff (O), Schwefel (S) oder $NR^5$, bevorzugt $CH_2$ und O,

Y:

- $N(R^{10})_2$, bevorzugt $NH_2$ und $N(CH_3)_2$,

- Hydroxy (OH),

- $C_{2-20}$-Alkylaminoalkyl, bevorzugt $C_{2-16}$-Alkylaminoalkyl, wie Methylaminomethyl, 2-Methylaminoethyl, Ethylaminomethyl, 2-Ethylaminoethyl und 2-(iso-Propylamino)ethyl,

- $C_{3-20}$-Dialkylaminoalkyl, bevorzugt $C_{3-16}$-Dialkylaminoalkyl, wie Dimethylaminomethyl, 2-Dimethylaminoethyl, 2-Diethylaminoethyl, 2-(Di-n-propylamino)ethyl und 2-(Di-isopropylamino)ethyl,

Z:

- $CH_2$, $CHR^5$, O, $NR^5$ oder $NCH_2CH_2OH$,

j, l:

- eine ganze Zahl von 1 bis 4 (1, 2, 3 oder 4), bevorzugt 2 und 3, besonders bevorzugt 2,

k, m, q:

- eine ganze Zahl von 1 bis 4 (1, 2, 3 oder 4), bevorzugt 2, 3 und 4, besonders bevorzugt 2 und 3,

n:

- eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Mit dem erfindungsgemäßen Verfahren können weiterhin Polyetheramine hergestellt werden. Bevorzugt können Polyetheramine der Formel IX

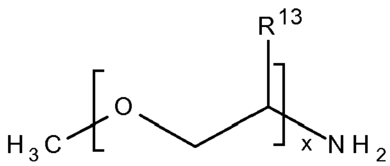

(IX)

in der $R^{13}$ H oder $CH_3$ bedeutet und x eine ganze Zahl von 1 bis 100, bevorzugt 2 bis 50, besonders bevorzugt 3 bis 20 bedeutet, oder
Polyetheramine der Formel X

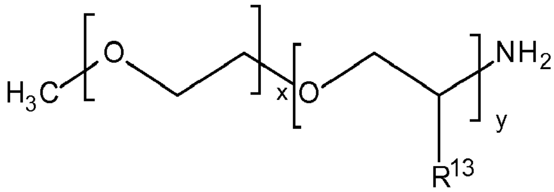

(X)

in der $R^{13}$ die oben genannten Bedeutung und x und y unabhängig voneinander eine ganze Zahl von 1 bis 100, bevorzugt 2 bis 50, besonders bevorzugt 3 bis 20 bedeutet, oder
Polyetheramine der Formel XI

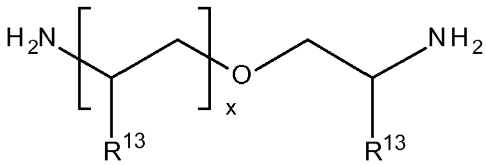

(XI)

In der $R^{13}$ und x die oben genannte Bedeutung hat, oder
Polyetheramine der Formel XII

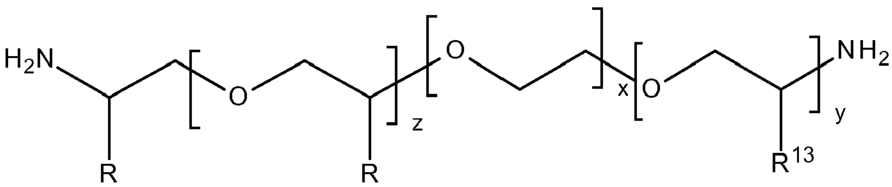

(XII)

in der $R^{13}$ die oben genannten Bedeutung und x, y und z unabhängig voneinander eine ganze Zahl von 1 bis 100, bevorzugt 2 bis 50, besonders bevorzugt 3 bis 20, oder
Polyetheramine der Formel XIII

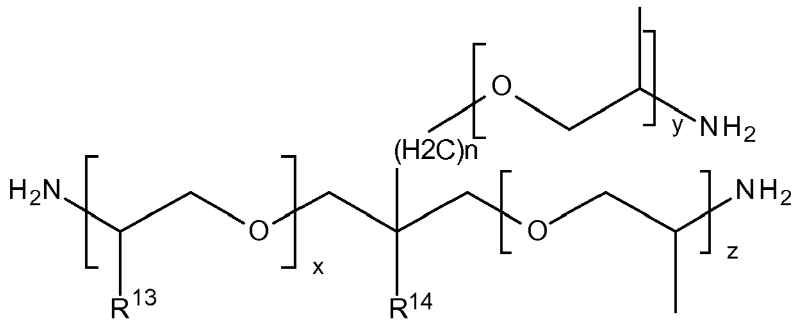
(XIII)

in der $R^{13}$, x, y und z die oben genannten Bedeutung, $R^{14}$ ein verzweigter oder unverzweigter Kohlenwasserstoffrest mit 1 bis 3 C Atomen ist und n entweder 0 oder 1 ist, oder
Polyetheramine der Formel XIV

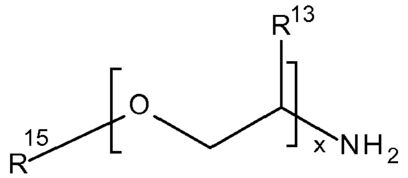
(XIV)

in der $R^{13}$ und x die oben genannten Bedeutung und $R^{15}$ ein verzweigter oder unverzweigter Kohlenwasserstoffrest mit 1 bis 30, bevorzugt 5 bis 20, besonders bevorzugt 10 bis 18 C-Atomen ist.

Ganz besonders bevorzugt Polyetherdiamine sind 4,7,10-Trioxatridecane-1,13-diamin, 4,9-Dioxadodecan-1,12-diamin und sogenannte Jeffamine® der Fa. Huntsman, insbesondere Jeffamin D230, Jeffamin D400, Jeffamin D2000, Jeffamin D4000, Jeffamin ED600, Jeffamin ED900, Jeffamin ED2003, Jeffamin EDR148 und Jeffamin EDR176 (Bezeichnungen aus der Produktbroschüre der Fa. Alfa Chemicals Ltd mit der Referenznummer "Hunt32").

Als Alkohole können unter den o.g. Voraussetzungen praktisch alle primären und sekundären Alkohole mit aliphatischer OH-Funktion eingesetzt werden. Die Alkohole können geradkettig, verzweigt oder zyklisch sein. Sekundäre Alkohole werden ebenso aminiert wie primäre Alkohole. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Alkohole, wie z. B. Diole oder Triole, besonders Glykole, aminiert werden, so hat man es über die Steuerung der Reaktionsbedingungen in der Hand, bevorzugt Aminoalkohole, zyklische Amine oder mehrfach aminierte Produkte zu erhalten.

Die Aminierung von 1,2-Diolen führt je nach Wahl der Reaktionsbedingungen besonders zu 1-Amino-2-hydroxy- oder 1,2-Diamino-Verbindungen.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidine).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethylenimine).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidine, 1,5-Di-piperidinyl-pentane).

Aus Diglykol (DEG) kann demnach durch Aminierung mit $NH_3$ Monoaminodiglykol (= ADG = $H_2N\text{-}CH_2CH_2\text{-}O\text{-}CH_2CH_2\text{-}OH$), Diaminodiglykol ($H_2N\text{-}CH_2CH_2\text{-}O\text{-}CH_2CH_2\text{-}NH_2$) oder Morpholin erhalten werden. Besonders bevorzugt ist hier das ADG als Verfahrensprodukt. Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:

Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclobutanol, Cyclopentanol, Cyclohexanol, Benzylalkohol, 2-Phenyl-ethanol, 2-(p-Methoxyphenyl)ethanol, 2-(3,4-Dimethoxyphenyl)ethanol, 1-Phenyl-3-butanol, Ethanolamin, n-Propanolamin, Isopropanolamin, 2-Amino-1-propanol, 1-Methoxy-2-propanol, 3-Amino-2,2-dimethyl-1-propanol, n_Pentanolamin (1-Amino-5-pentanol), n-Hexanolamin (1-Amino-6-hexanol), Ethanolamin, Diethanolamin, Triethanolamin, N-Alkyldiethanolamine, Diisopropanolamin, 3-(2-Hydroxy-ethylamino)propan-1-ol, 2-(N,N-Dimethylamino)ethanol, 2-(N,N-Diethylamino)ethanol, 2-(N,N-Di-n-propylamino)ethanol, 2-(N,N-Di-iso-propylamino)ethanol, 2-(N,N-Di-n-butylamino)ethanol, 2-(N,N-Di-iso-butylamino)ethanol, 2-(N,N-Di-sec.-butylamino)ethanol, 2-(N,N-Di-tert.-butylamino)ethanol, 3-(N,N-Dimethylamino)propanol, 3-(N,N-Diethylamino)propanol, 3-(N,N-Di-n-propylamino)propanol, 3-(N,N-Di-iso-propylamino)propanol, 3-(N,N-Di-n-butylamino)propanol, 3-(N,N-Di-iso-butylamino)propanol, 3-(N,N-Di-sec.-butylamino)propanol, 3-(N,N-Di-tert.-butylamino)propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Besonders bevorzugte Alkohole sind Methanol, Ethanol, n-Propanol, i-Propanol, n-Butanol, sek.-Butanol, 1,4-Butandiol, 1,5-Pentandiol, 1,6-Hexandiol, 2-Ethylhexanol, Cyclohexanol, Fettalkohole, Ethylenglykol, Diethylenglykol (DEG), Triethylenglykol (TEG), 2-(2-Dimethylamino-ethoxy)ethanol, N-Methyldiethanolamin und 2-(2-Dimethylaminoethoxy)ethanol.

Weiterhin werden bevorzugt Polyetherole in das Verfahren eingesetzt.

Besonders bevorzugte Polyetherole sind

Polyetherole der Formel XV

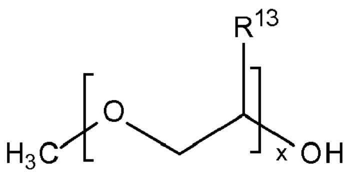

(XV)

in der $R^{13}$ H oder $CH_3$ bedeutet und x eine ganze Zahl von 1 bis 100, bevorzugt 2 bis 50, besonders bevorzugt 3 bis 20 bedeutet, oder

Polyetherole der Formel XVI

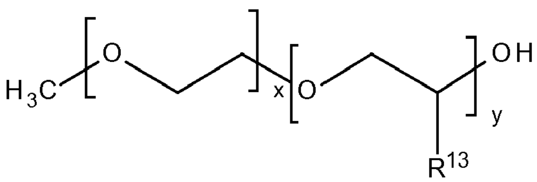

(XVI)

in der $R^{13}$ die oben genannten Bedeutung und x und y unabhängig voneinander eine ganze Zahl von 1 bis 100, bevorzugt 2 bis 50, besonders bevorzugt 3 bis 20 bedeutet, oder

Polyetherole der Formel XVII

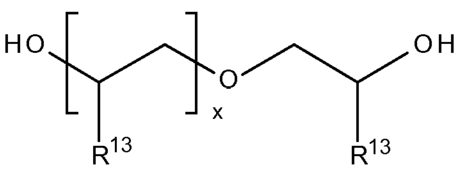

(XVII)

In der $R^{13}$ und x die oben genannte Bedeutung hat, oder

Polyetheramine der Formel XVIII

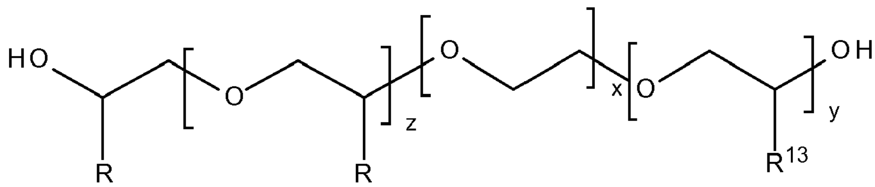

(XVIII)

in der $R^{13}$ die oben genannten Bedeutung und x, y und z unabhängig voneinander eine ganze Zahl von 1 bis 100, bevorzugt 2 bis 50, besonders bevorzugt 3 bis 20, oder
Polyetheramine der Formel XIX

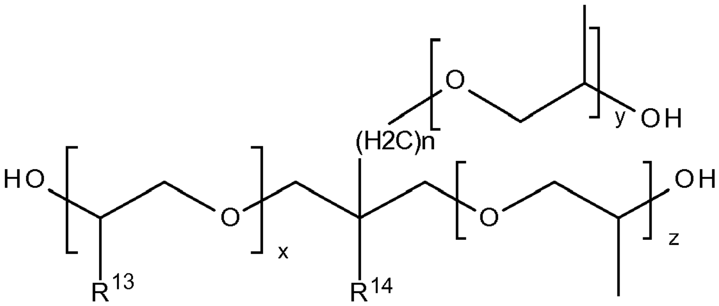

(XIX)

in der $R^{13}$, x, y und z die oben genannten Bedeutung, $R^{14}$ ein verzweigter oder unverzweigter Kohlenwasserstoffrest mit 1 bis 3 C Atomen ist und n entweder 0 oder 1 ist, oder
Polyetheramine der Formel XX

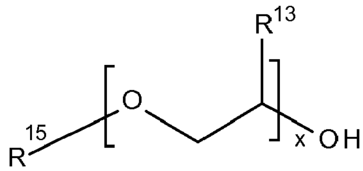

(XX)

in der $R^{13}$ und x die oben genannten Bedeutung und $R^{15}$ ein verzweigter oder unverzweigter Kohlenwasserstoffrest mit 1 bis 30, bevorzugt 5 bis 20, besonders bevorzugt 10 bis 18 C-Atomen.

In das erfindungsgemäße Verfahren können unter den o.g. Voraussetzungen praktisch alle aliphatischen und aromatischen Ketone eingesetzt werden. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, Butanon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

In das erfindungsgemäße Verfahren können unter den o.g. Voraussetzungen praktisch alle aliphatischen und aromatischen Aldehyde eingesetzt werden. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)acetaldehyd, 4-Formyltetrahydropyran, 3- Formyltetrahydrofuran, 5-Formylvaleronitril, Citronellal, Lysmeral,

Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Alkoholen, Aldehyden oder Ketonen in Gegenwart von Wasserstoff können sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe bzw. die Aldehydgruppe bzw. die Ketogruppe zunächst in die primäre Aminogruppen ($-NH_2$) umgewandelt. Das so gebildete primäre Amin können mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise) und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Aus mehrwertigen Alkoholen bzw. Di- oder Oligoaldehyden bzw. Di- oder Oligoketonen bzw. Ketoaldehyden lassen sich auf diese Weise durch intramolekulare hydrierende Aminierung zyklische Amine wie z.B. Pyrrolidine, Piperidine, Hexamethylenimine, Piperazine und Morpholine herstellen.

Bevorzugt wird in das Verfahren reiner Ammoniak eingesetzt, bevorzugt Ammoniak mit einem Gehalt von mehr als 99 Gew.-% Ammoniak und besonders bevorzugt mehr als 99,9 Gew.-% Ammoniak

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Beispielsweise werden die folgenden Mono- und Dialkylamine als Aminierungsmittel verwendet: Monomethylamin, Dimethylamin, Monoethylamin, Diethylamin, n-Propylamin, Di-n-propylamin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Bevorzugte Aminierungsmittel sind Ammoniak, Monomethylamin, Dimethylamin, Monoethylamin, Diethylamin, n-Propylamin, Di-n-propyl-amin, iso-Propylamin und Di-isopropyl-amin.

Ganz besonders bevorzugt wird Ammoniak als Aminierungsmittel eingesetzt.

Mit dem erfindungsgemäßen Verfahren besonders bevorzugt hergestellte Amine sind zum Beispiel Morpholin (aus Monoaminodiglykol), Monoaminodiglykol, Morpholin und/oder 2,2'-Dimorpholinodiethylether (DMDEE) (aus DEG und Ammoniak), 6-Dimethylaminohexanol-1 (aus Hexandiol und Dimethylamin (DMA)), Triethylamin (aus Ethanol und Diethylamin (DEA)), Dimethylethylamin (aus Ethanol und DMA), N-($C_{1-4}$-alkyl)morpholin (aus DEG und Mono($C_{1-4}$-alkyl)amin), N-($C_{1-4}$-alkyl)piperidin (aus 1,5-Pentandiol und Mono($C_{1-4}$-alkyl)amin), Piperazin und/oder Diethylentriamin (DETA) (aus N-(2-Aminoethyl)-ethanolamin (AEEA) und Ammoniak), N-Methylpiperazin (aus Diethanolamin und MMA), N,N'-Dimethylpiperazin (aus N-Methyldiethanolamin und MMA), 1,2-Ethylendiamin (EDA) und/oder Diethylentriamin (DE-TA) und/oder PIP (aus Monoethanolamin (MEOA) und Ammoniak), 2-Ethylhexylamin und Bis(2-Ethylhexyl)amin (aus 2-Ethylhexanol und $NH_3$), Tridecylamin und Bis(Tridecyl)amin (aus Tridecanol und $NH_3$), n-Octylamin (aus n-Octanol und $NH_3$), 1,2-Propylendiamin (aus 2-Hydroxy-propylamin und $NH_3$), 1-Diethylamino-4-aminopentan (aus 1-Diethylamino-4-hydroxypentan und $NH_3$), N,N-Di($C_{1-4}$-alkyl)cyclohexylamin (aus Cyclohexanon und/oder Cyclohexanol und Di($C_{1-4}$-alkyl)amin), z.B. N,N-Dimethyl-N-cyclohexylamin (DMCHA), Polyisobutenamin (PIBA; mit z.B. n~1000) (aus Polyisobutenaldehyd und $NH_3$), N,N-Diisopropyl-N-ethylamin (Hünigbase) (aus N,N-Diisopropylamin und Acetaldehyd), N-Methyl-N-iso-propylamin (MMIPA) (aus Monomethylamin und Aceton), n-Propylamine (wie Mono-/Di-n-propylamin, N,N-Dimethyl-N-n-propylamin (DMPA)) (aus Propionaldehyd und/oder n-Propanol und $NH_3$ bzw. DMA), N,N-Dimethyl-N-isopropylamin (DMIPA) (aus i-Propanol und/oder Aceton und DMA), N,N-Dimethyl-N-butylamine (1-, 2- oder iso-Butanol und/oder Butanal, i-Butanal oder Butanon und DMA), 2-(2-Di($C_{1-4}$-alkyl)aminoethoxy)ethanol und/oder Bis(2-di($C_{1-4}$-alkyl)aminoethyl)ether (aus DEG und Di($C_{1-4}$-alkyl)amin), 1,2-Ethylendiamin (EDA), Monoethanolamin (MEOA), Diethylentriamin (DETA) und/oder Piperazin (PIP) (aus Monoethylenglykol (MEG) und Ammoniak), 1,8-Diamino-3,6-dioxa-octan und/oder 1-Amino-8-hydroxy-3,6-dioxa-octan (aus Triethylenglykol (TEG) und Ammoniak), 1-Methoxy-2-propylamin (1-Methoxy-isopropylamin, MOIPA) (aus 1-Methoxy-2-propanol und Ammoniak), N-Cyclododecyl-2,6-dimethylmorpholin (Dodemorph) (aus Cyclododecanon und/oder Cyclododecanol und 2,6-Dimethylmorpholin), Polyetheramin (aus entsprechendem Polyetheralkohol und Ammoniak). Die Polyetheralkohole sind z.B. Polyethylenglykole oder Polypropylenglykole mit einem Molekulargewicht im Bereich von 200 bis 5000 g/mol, die entsprechende Polyetheramine sind z.B. unter dem Handelsname PEA D230, D400, D2000, T403 oder T5000 von BASF erhältlich.

Die Umsetzung der Alkohole, Aldehyde und/oder Ketone erfolgt in Gegenwart von Wasserstoff. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum

Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Die Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, erfolgt in Gegenwart eines Katalysators.

Als Katalysatoren können insbesondere Katalysatoren eingesetzt werden, die als aktive Komponente ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Eine weitere bevorzugte aktive Komponente ist Cu.

Die oben genannten Katalysatoren können in üblicher Weise mit Promotoren, beispielsweise mit Chrom, Eisen, Kobalt, Mangan, Molybdän, Titan, Zinn, Metallen der Alkaligruppe, Metallen der Erdalkaligruppe und/oder Phosphor dotiert werden.

Als Katalysatoren werden bevorzugt sogenannte Skelett-Katalysatoren (auch als Raney®-Typ bezeichnet, nachfolgend auch: Raney-Katalysator) eingesetzt werden, die durch Auslaugen (Aktivierung) einer Legierung aus hydrieraktivem Metall und einer weiteren Komponente (bevorzugt Al) erhalten werden. Bevorzugt werden Raney-Nickel-Katalysatoren oder Raney-Cobalt-Katalysatoren eingesetzt.

Als Katalysatoren werden weiterhin bevorzugt Pd oder Pt-Trägerkatalysatoren eingesetzt. Bevorzugte Trägermaterialien sind Aktivkohle, $Al_2O_3$, $TiO_2$, $ZrO_2$ und $SiO_2$.

In einer weiteren bevorzugten Ausführungsform werden in das erfindungsgemäße Verfahren Katalysatoren eingesetzt, die durch Reduktion von sogenannten Katalysatorvorläufern hergestellt werden.

Der Katalysatorvorläufer enthält eine aktive Masse, die eine oder mehrere katalytisch aktive Komponenten, ggf. Promotoren und optional ein Trägermaterial enthält.

Bei den katalytisch aktiven Komponenten handelt es sich um sauerstoffhaltige Verbindungen der oben genannten Metalle, beispielsweise um deren Metalloxide bzw. Hydroxide, wie CoO, NiO, CuO und/oder deren Mischoxide.

Im Rahmen dieser Anmeldung wird der Begriff katalytisch aktive Komponenten für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.

Besonders bevorzugt sind Katalysatorvorläufer, wie die

in EP-A-0636409 offenbarten Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,2 bis 5,0 Gew.-% Alkali, berechnet als $M_2O$ (M=Alkali), enthalten, oder

in EP-A-0742045 offenbarte Oxidgemische, die vor der Reduktion mit Wasserstoff 55 bis 98 Gew.-% Co, berechnet als CoO, 0,2 bis 15 Gew.-% Phosphor, berechnet als $H_3PO_4$, 0,2 bis 15 Gew.-% Mangan, berechnet als $MnO_2$, und 0,05 bis 5 Gew.-% Alkali, berechnet als $M_2O$ (M=Alkali), enthalten, oder

in EP-A-696572 offenbarte Oxidgemische, die vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% sauerstoffhaltige Verbindungen des Aluminiums und/oder Mangans, berechnet als $Al_2O_3$ bzw. $MnO_2$, enthält, beispielsweise der in loc. cit, Seite 8, offenbarte Katalysator mit der Zusammensetzung 31,5 Gew.-% $ZrO_2$, 50 Gew.-% NiO, 17 Gew.-% CuO und 1,5 Gew.-% $MoO_3$, enthalten oder

in EP-A-963 975 offenbarte Oxidgemische, die vor der Reduktion mit Wasserstoff 22 bis 40 Gew.-% $ZrO_2$, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als $Al_2O_3$ bzw. $MnO_2$, und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als $ZrO_2$, 28 Gew.-% Ni, berechnet als NiO, 11 Gew.-% Cu, berechnet als CuO und 28 Gew.-% Co, berechnet als CoO, enthalten.

Die Katalysatoren bzw. Katalysatorvorläufer werden bevorzugt in Form von Formkörpern in das erfindungsgemäße Verfahren eingesetzt.

Als Formkörper eignen sich Formkörper mit beliebiger Geometrie bzw. Form. Bevorzugte Formen sind Tabletten, Ringe, Zylinder, Sternstränge, Wagenräder oder Kugeln, besonders bevorzugt sind Tabletten, Ringe, Zylinder, Kugeln oder Sternstränge. Ganz besonders bevorzugt ist die Strangform.

Bei Kugeln beträgt der Durchmesser der Kugelform bevorzugt 10 mm oder weniger, besonders bevorzugt 4

mm oder weniger, ganz besonders bevorzugt 3 mm oder weniger und insbesondere bevorzugt 2,5 mm oder weniger.

In einer bevorzugten Ausführungsform liegt bei Kugeln der Durchmesser der Kugelform bevorzugt im Bereich von 0,1 bis 10, besonders bevorzugt 0,5 bis 4 mm, ganz besonders bevorzugt 1 bis 3 mm und insbesondere besonders bevorzugt 1,5 bis 2,5 mm.

Bei Strängen oder Zylindern liegt das Verhältnis von Länge : Durchmessers bevorzugt im Bereich von 1:1 bis 20:1, besonders bevorzugt 1:1 bis 14:1, ganz besonders bevorzugt im Bereich von 1:1 1 bis 10:1 und insbesondere bevorzugt im Bereich von 1:2 bis 6:1.

Der Durchmesser der Stränge oder Zylinder beträgt bevorzugt 10 mm oder weniger, besonders bevorzugt 5 mm oder weniger, ganz besonders bevorzugt 3 mm oder weniger und insbesondere bevorzugt 2,5 mm oder weniger.

In einer bevorzugten Ausführungsform liegt der Durchmesser der Stränge oder Zylinder vorzugsweise im Bereich von 0,1 bis 10 mm, besonders bevorzugt im Bereich von 0,5 bis 3 mm, ganz besonders bevorzugt im Bereich von 1 bis 2,5 mm und insbesondere bevorzugt im Bereich von 1,5 bis 2,5 mm.

Bei Tabletten beträgt die Höhe h der Tablette vorzugsweise 10 mm oder weniger, besonders bevorzugt 4 mm oder weniger, ganz besonders bevorzugt 3 mm oder weniger und insbesondere bevorzugt 2,5 mm oder weniger.

In einer bevorzugten Ausführungsform liegt die Höhe h der Tablette bevorzugt im Bereich von 0,1 bis 10 mm, besonders bevorzugt im Bereich von 0,5 bis 4 mm, ganz besonders bevorzugt im Bereich von 1 bis 3 mm und insbesondere bevorzugt im Bereich von 1,5 bis 2,5 mm.

Das Verhältnis von Höhe h (bzw. Dicke) der Tablette zum Durchmesser D der Tablette beträgt bevorzugt 1:1 bis 1:5, besonders bevorzugt 1:1 bis 1:2,5, ganz besonders bevorzugt 1:1 bis 1:2 und insbesondere bevorzugt 1:1 bis 1:2.

Bei allen anderen Geometrien weist der Katalysatorformkörper im erfindungsgemäßen Verfahren jeweils bevorzugt einen Äquivalentdurchmesser L = 1/a’ von 2 mm oder weniger, besonders bevorzugt 1 mm oder weniger, ganz besonders bevorzugt 0,7 mm oder weniger und insbesondere bevorzugt 0,5 mm oder weniger auf, wobei a’ die externe Oberfläche per Volumeneinheit ($mms^2/mmp^3$) ist, mit:

$$a' = \frac{A_p}{V_p},$$

wobei Ap die externe Oberfläche des Formkörpers ($mm_s^2$) und Vp das Volumen des Formkörpers ($mm_p^3$) ist.

In einer bevorzugten Ausführungsform weist der Katalysatorformkörper im erfindungsgemäßen Verfahren bei allen anderen Geometrien jeweils bevorzugt einen Äquivalentdurchmesser L = 1/a’ im Bereich von 0,1 bis 2mm, besonders bevorzugt im Bereich von 0,1 bis 0,7 mm, ganz besonders bevorzugt im Bereich von 0,2 bis 0,5 mm und insbesondere bevorzugt im Bereich von 0,3 bis 0,4 mm auf.

Die Oberfläche und das Volumen des Formkörpers ergeben sich aus den geometrischen Abmessungen des Formkörpers gemäß den bekannten mathematischen Formeln.

Das Volumen kann auch nach folgender Methode berechnet werden, bei der man:

1. Die innere Porosität des Formkörpers bestimmt (z.B. über Messung der Wasseraufnahme in [ml/g Kat] bei Raumtemperatur und 1 bar Gesamtdruck),
2. die Verdrängung des Formkörpers beim Eintauchen in eine Flüssigkeit (z.B. durch Gasverdrängung mittels Helium-Pyknometer) bestimmt und
3. die Summe beider Volumina bildet.

Die Oberfläche kann auch nach folgender Methode theoretisch berechnet werden, bei der man eine Umhüllende des Formkörpers definiert, deren Kurvenradien max. 5 $\mu$m beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Der eingesetzte Formkörper weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l

In einer bevorzugten Ausführungsform werden die Katalysatoren in Form von Formkörpern in das erfindungsgemäße Verfahren eingesetzt, die durch Tränkung (Imprägnierung) von Trägermaterialien hergestellt werden, die oben genannte Geometrie aufweisen oder die nach der Tränkung zur Formkörpern, die die oben genannte Geometrie aufweisen verformt werden.

Als Trägermaterialien kommen beispielsweise Kohlenstoff, wie Graphit, Ruß, Graphen, Kohlenstoffnanoröhrchen und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Calcinierung wird im Allgemeinen bei Temperaturen zwischen 300 und 800°C, vorzugsweise 350 bis 600°C, insbesondere bei 450 bis 550°C ausgeführt.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

Bevorzugt werden Trägermaterialien eingesetzt, die bereits die zu vor beschriebene bevorzugte Geometrie der Formkörper aufweisen.

Es ist jedoch auch möglich Trägermaterialien einzusetzen, die als Pulver oder Splitt vorliegen, und getränkten Trägermaterialien einer Formgebung zu unterziehen.

So kann beispielsweise das imprägnierte und getrocknete bzw. calcinierten Trägermaterial konditioniert werden.

Die Konditionierung kann beispielsweise dadurch erfolgen, dass man das getränkte Trägermaterial durch Vermahlen auf eine bestimmte Korngröße einstellt.

Nach der Vermahlung kann das konditioniete, getränkte Trägermaterial mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen. Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

In einer bevorzugten Ausführungsform werden Formkörper in das erfindungsgemäße Verfahren eingesetzt, die durch eine gemeinsame Fällung (Mischfällung) aller ihrer Komponenten hergestellt werden und die so ausgefällten Katalysatorvorläufer einer Formgebung unterzogen werden.

Dazu wird in der Regel eine lösliche Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

In einer weiteren, bevorzugten Ausführungsform können die Formkörper durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Üblicherweise werden bei den Fällungsreaktionen die löslichen Verbindungen durch Zugabe eines Fällungsmittels als schwer- oder unlösliche, basische Salze gefällt.
Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.
Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100°C, besonders 30 bis 90°C, insbesondere bei 50 bis 70°C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 350 bis 600°C, insbesondere bei 450 bis 550°C ausgeführt.

Nach der Calcinierung werden die durch Fällungsreaktionen erhaltenen pulverförmigen Katalysatorvorläufer üblicherweise konditioniert.
Die Konditionierung kann beispielsweise dadurch erfolgen, dass man den Fällungskatalysator durch Vermahlen auf eine bestimmte Korngröße einstellt.
Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.
Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

Formkörper, die die durch Tränkung oder Fällung hergestellt wurden, enthalten in der Regel die katalytisch aktiven Komponenten nach erfolgter Calcinierung in der Regel in Form ihrer sauerstoffhaltige Verbindungen, beispielsweise um deren Metalloxide bzw. Hydroxide, wie CoO, NiO, CuO und/oder deren Mischoxide (Katalysatorvorläufer).

Die Katalysatorvorläufer, die wie voranstehend beschrieben durch Imprägnierung oder Fällung hergestellt wurden, werden im Allgemeinen nach der Calcinierung bzw. Konditionierung reduziert. Durch die Reduktion wird der Katalysatorvorläufer in der Regel in seine katalytisch aktive Form umgewandelt.

Die Reduktion des Katalysatorvorläufers kann bei erhöhter Temperatur in einem bewegten oder unbewegten Reduktionsofen durchgeführt werden.
Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, ggf. vermischt mit Frisch-Wasserstoff und ggf. nach Entfernen von Wasser durch Kondensation. Die Reduktion des Katalysatorvorläufers erfolgt bevorzugt in einem Reaktor, in dem die Formkörper als Festbett angeordnet sind. Besonders bevorzugt erfolgt die Reduktion des Katalysatorvorläufers in demselben Reaktor in dem die nachfolgende Umsetzung der Alkohle, Aldehyde und/oder Ketone mit Wasserstoff erfolgt.

Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600°C, insbesondere von 100 bis 500°C, besonders bevorzugt von 150 bis 450°C.
Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der so erhaltene Formkörper kann nach der Reduktion unter inerten Bedingungen gehandhabt werden. Bevorzugt kann der Formkörper unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.

Die Lagerung des Katalysators unter inerten Substanzen ermöglicht eine unkomplizierte und ungefährliche Handhabung und Lagerung des Formkörpers.

Der Formkörper kann nach der Reduktion aber auch mit einem Sauerstoff enthaltenden Gasstrom wie Luft oder einem Gemisch von Luft mit Stickstoff in Kontakt gebracht werden. Dadurch wird ein passivierter Formkörper erhalten. Der passivierte Formkörper weist im Allgemeinen eine schützende Oxidschicht auf. Durch diese schützende Oxidschicht wird die Handhabung und Lagerung des Katalysators vereinfacht, so dass beispielsweise der Einbau des passivierten Formkörpers in den Reaktor vereinfacht wird. Ein passivierter Formkörper wird bevorzugt vor dem Inkontakt bringen mit den Edukten wie oben beschrieben durch Behandlung des passivierten Katalysators mit Wasserstoff oder einem Wasserstoff enthaltenden Gas reduziert. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben.

Das erfindungsgemäße Verfahren wird bevorzugt in einem Reaktor durchgeführt, in dem der Katalysator als Festbett angeordnet ist.

In einer bevorzugten Ausführungsform umfasst die Festbettanordnung eine Katalysatorschüttung im eigentlichen Sinn, d. h. lose, geträgerten oder ungeträgerte Formkörper, die bevorzugt in der zuvor beschriebenen Geometrie oder Form vorliegen.

Dazu werden die Formkörper in den Reaktor eingebracht.

Damit die Formkörper in dem Reaktor verbleiben und nicht durch diesen hindurchfallen, wird üblicherweise ein Gitterboden oder ein gas- und flüssigkeitsdurchlässiges Blech eingesetzt, auf dem die Formkörper aufliegen.

Die Formkörper können sowohl am Eingang als am Ausgang des Reaktors von einem Inertmaterial umgeben sein. Als Inertmaterial werden in der Regel Formkörper eingesetzt, die eine ähnliche Geometrie aufweisen, wie die zuvor beschriebenen Katalysatorformköprer, sich jedoch in der Reaktion inert verhalten, z.B. Pallringe, Kugeln aus einem inerten Material (z.B. Keramik, Steatit, Aluminium).

Die Formkörper können aber auch mit Inertmaterial durchmischt werden und als Mischung in den Reaktor eingebracht werden.

Die Katalysatorschüttung (Formkörper + ggf. Inertmaterial) weist bevorzugt eine Schüttdichte (nach EN ISO 6) im Bereich von 0,1 bis 3 kg/l, bevorzugt von 1,5 bis 2,5 kg/l und insbesondere bevorzugt 1,7 bis 2,2 kg/l.

Der Differenzdruck über die Schüttung beträgt bevorzugt weniger als 1000 mbar/m, bevorzugt weniger als 800 mbar/m und besonders bevorzugt weniger als 700 mbar/m. Bevorzugt liegt der Differenzdruck über die Schüttung im Bereich von 10 bis 1000 mbar/m, bevorzugt 50 bis 800 mbar/m, besonders bevorzugt 100 bis 700 mbar/m und insbesondere im Bereich von 200 bis 500 mbar/m.

Bei Rieselfahrweise (Durchflussrichtung der Flüssigkeit von oben nach unten) ergibt sich der Differenzdruck aus dem oberhalb der Katalysatorschüttung gemessenen Druck und dem unterhalb der Katalysatorschüttung gemessenen Druck.

Bei Sumpffahrweise (Durchflussrichtung der Flüssigkeit von unten nach oben) ergibt sich der Differenzdruck aus dem unterhalb der Katalysatorschüttung gemessenen Druck und dem oberhalb der Katalysatorschüttung gemessenen Druck.

Geeignete Festbettreaktoren sind beispielsweise in dem Artikel "Fixed-Bed Reactors" (Ullmann's Encyclopedia of Industrial Chemistry, Published Online: 15 JUN 2000, DOI: 10.1002/14356007.b04_199) beschrieben.

Bevorzugt wird das Verfahren in einem Schachtreaktor, Rohrbündelreaktor oder Rohrreaktor durchgeführt.

Besonders bevorzugt wird das Verfahren in einem Rohrreaktor durchgeführt.

Die Reaktoren können jeweils als einzelner Reaktor, als Serie von einzelnen Reaktoren und/oder in Form von zwei oder mehr parallelen Reaktoren eingesetzt werden.

Der spezielle Reaktoraufbau und die Durchführung der Reaktion können in Abhängigkeit von dem durchzuführenden Verfahren, den erforderlichen Reaktionszeiten und der Natur des eingesetzten Katalysators variieren.

Bevorzugt beträgt das Verhältnis von Höhe zu Durchmesser des Reaktors, insbesondere eines Rohrreaktors,

1:1 bis 500:1, besonders bevorzugt 2:1 bis 100:1 und insbesondere bevorzugt 5:1 bis 50:1.

Die Fließrichtung der Reaktanden (Edukte, Wasserstoff, ggf. flüssiger Ammoniak) ist in der Regel von oben nach unten bzw. von unten nach oben.

Besonders bevorzugt ist die Fließrichtung der Reaktanden (Edukte, Wasserstoff, ggf. flüssiger Ammoniak) von oben nach unten durch den Reaktor.

Die Katalysatorbelastung bei kontinuierlicher Fahrweise liegt typischerweise bei 0,01 bis 10, vorzugsweise von 0,2 bis 5, besonders bevorzugt von 0,2 bis 4 kg Edukt pro L Katalysator und Stunde.

Die Querschnittsbelastung liegt erfindungsgemäß im Bereich von 5 kg/($m^2$ s) bis 50 kg/($m^2$s), bevorzugt 8 bis 25 kg/($m^2$ s), besonders bevorzugt 10 bis 20 kg/($m^2$ s) und insbesondere bevorzugt 12 bis 18 kg/($m^2$ s).

Die Querschnittsbelastung v [kg/($m^2$ s)] ist definiert als

$$v = \frac{Q}{A},$$

wobei Q die Durchflussmasse [kg/s] und A die Querschittsfläche der leeren Kolonne ist [$m^2$]. Die Durchflussmasse Q ist wiederum definiert als die Summe der Massen aller zugeführten Eduktströme und Rückführströme. Wasserstoff, Kreisgase und eventuell zugeführte Inertgase werden nicht zur Berechnung der Durchflussmasse verwendet, da Wasserstoff, Kreisgase und Inertgase bei den üblichen Hydrierbedingungen in der Regel in der Gasphase vorliegen.

Um die hohen Querschnittsbelastungen zu erzielen, wird bevorzugt ein Teil des Austrags (Teilaustrag) aus dem Hydrierreaktor als Rückführstrom in den Reaktor zurückgeführt (Umlaufstrom). Der Umlaufstrom kann dem Reaktor separat zugeführt werden oder er kann besonders bevorzugt mit den zugeführten Edukten vermischt werden und zusammen mit diesem dem Reaktor wieder zugeführt werden.

Das Verhältnis von Umlaufstrom zum zugeführten Eduktstrom liegt bevorzugt im Bereich von 0,5:1 bis 250:1, besonders bevorzugt im Bereich von 1:1 bis 200:1, und insbesondere bevorzugt im Bereich von 2:1 bis 180:1. Wenn in das Verfahren kein Ammoniak zugeführt wird, dann liegt das Verhältnis von Umlaufstrom zum zugeführten Eduktstrom bevorzugt im oberen Bereich der voranstehend genannten Bereiche. Wenn hingegen in das Verfahren viel Ammoniak zugeführt wird, dann liegt das Verhältnis von Umlaufstrom zum zugeführten Eduktstrom bevorzugt im unteren Bereich der voranstehend genannten Bereiche.

In einer weiteren bevorzugten Ausführungsform können hohe Querschnittsbelastungen erzielt werden, wenn die Reaktion in einem Reaktor mit schlanker Bauweise, insbesondere in einem Rohrreaktor mit schlanker Bauweise, durchgeführt wird.

Das Verhältnis von Höhe zu Durchmesser des Reaktors liegt deshalb, wie voranstehend beschrieben, bevorzugt im Bereich von 1:1 bis 500:1, besonders bevorzugt im Bereich von 2:1 bis 100:1 und insbesondere bevorzugt im Bereich von 5:1 bis 50:1.

Die Umsetzung wird in der Regel bei einem Druck von 1 bis 200 bar, insbesondere von 5 bis 150 bar, bevorzugt von 10 bis 100 bar und besonders bevorzugt von 15 bis 95 bar durchgeführt. Ganz besonders bevorzugt wird die Umsetzung bei einem Druck von weniger als 95 bar als Niederdruckverfahren ausgeführt.

Die Temperatur liegt in der Regel in einem Bereich 25 bis 300°C, insbesondere von 50 bis 200°C, bevorzugt von 70 bis 150°C, besonders bevorzugt von 80 bis 140°C.

Dabei werden die Reaktionsbedingungen bevorzugt so gewählt, dass die eingesetzten Einsatzstoffe und ggf. zugegebene Flüssigkeiten in der Regel in der Flüssigphase vorliegen und nur der eingesetzte Wasserstoff bzw. Inertgase unter den genannten Reaktionsbedingungen in der Gasphase vorliegen.

Bevorzugt wird im Falle der Aminierung von Alkoholen, Aldehyden oder Ketonen mit primären oder sekundären Aminen das Amin in ca. stöchiometrischer Menge oder geringfügig überstöchiometrischer Menge pro Mol zu aminierender alkoholischer Hydroxylgruppe, Aldehydgruppe oder Ketogruppe eingesetzt.

Die Aminkomponente (Stickstoffverbindung) wird bevorzugt in der 0,90- bis 100-fachen molaren Menge, insbesondere in der 1,0- bis 10-fachen molaren Menge, jeweils bezogen auf den/das eingesetzte/n Alkohol, Aldehyd und/oder Keton eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe, Aldehydgruppe oder Ketogruppe eingesetzt.

Höhere Überschüsse sowohl an Ammoniak als auch an primären oder sekundären Aminen sind möglich.

Der Wasserstoff wird der Reaktion im allgemeinen in einer Menge von 5 bis 400 l, bevorzugt in einer Menge von 50 bis 200 l pro Mol Alkohol-, Aldehyd- oder Ketonkomponente zugeführt, wobei die Literangaben jeweils auf Normalbedingungen umgerechnet wurden (S.T.P.). Die Aminierung von Aldehyden bzw. Ketonen unterscheidet sich in der Durchführung von der Aminierung von Alkoholen dadurch, dass bei der Aminierung von Aldehyden und Ketonen

mindestens stöchiometrische Mengen an Wasserstoff vorhanden sein müssen.

Die Reaktion kann in Substanz oder in einer Flüssigkeit durchgeführt werden.
Die Umsetzung erfolgt bevorzugt in Gegenwart einer Flüssigkeit.

Geeignete Flüssigkeiten sind beispielsweise C1- bis C4-Alkohole, wie Methanol oder Ethanol, C4- bis C12-Dialkylether, wie Diethylether oder tert-Butylmethylether, oder cyclische C4-bis C12-Ether, wie Tetrahydrofuran oder Dioxan, oder Kohlenwasserstoffe, wie Pentan, Hexan, Heptan, Octan, Cyclohexan oder Toluol. Geeignete Flüssigkeiten können auch Mischungen der vorstehend genannten Flüssigkeiten sein. In einer bevorzugten Ausführungsform ist die Flüssigkeit ein Produkt der Umsetzung.

Das im Zuge der Umsetzung gebildete Reaktionswasser (jeweils ein Mol pro Mol umgesetzte Alkoholgruppe, Aldehydgruppe bzw. Ketogruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Die Reaktion kann deshalb auch in Gegenwart von Wasser erfolgen. Der Wassergehalt sollte allerdings nicht mehr als 10 Gew.-%, vorzugsweise weniger als 5 Gew.-%, besonders bevorzugt weniger als 3 Gew.-% betragen, bezogen auf die Masse der eingesetzten Flüssigkeit, um das Auslaugen und/oder Abwaschen der Verbindungen der Alkali-, Erdalkali- und/oder Seltenen Erdmetalle weitestgehend zu vermeiden.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z.B. durch eine fraktionierende Rektifikation. Geeignete Aufarbeitungsverfahren sind z.B. in EP 1 312 600 A und EP 1 312 599 A (beide BASF AG) beschrieben. Das überschüssige Aminierungsmittel und der Wasserstoff werden vorteilhaft wieder in die Reaktionszone zurückgeführt. Das gleiche gilt für die eventuell nicht vollständig umgesetzte Alkohol-, Aldehyd- bzw. Ketonkomponente.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Die Aktivität und/oder Selektivität der erfindungsgemäßen Katalysatoren kann mit zunehmender Standzeit abnehmen. Demgemäß wurde ein Verfahren zur Regenerierung der erfindungsgemäßen Katalysatoren gefunden, bei dem man den Katalysator mit einer Flüssigkeit behandelt. Die Behandlung des Katalysators mit einer Flüssigkeit soll dazu führen, dass eventuell anhaftende Verbindungen, die aktive Stellen des Katalysators blockieren, abgelöst werden. Die Behandlung des Katalysators mit einer Flüssigkeit kann durch Rühren des Katalysators in einer Flüssigkeit oder durch Waschen des Katalysators in der Flüssigkeit erfolgen, wobei nach erfolgter Behandlung die Flüssigkeit durch Filtration oder Abdekantieren zusammen mit den abgelösten Verunreinigungen vom Katalysator abgetrennt werden kann.

Geeignete Flüssigkeiten sind in der Regel das Produkt der Umsetzung, Wasser oder ein organisches Lösungsmittel, bevorzugt Ether, Alkohole oder Amide.

In einer weiteren Ausführungsform kann die Behandlung des Katalysators mit Flüssigkeit in Gegenwart von Wasserstoff oder eines Wasserstoff enthaltenden Gases erfolgen.

Diese Regenerierung kann unter erhöhter Temperatur, in der Regel von 20 bis 250°C, durchgeführt werden. Es ist auch möglich, den gebrauchten Katalysator zu trocknen und anhaftende organische Verbindungen mit Luft zu flüchtigen Verbindungen wie $CO_2$ zu oxidieren. Vor einer weiteren Verwendung des Katalysators in der Umsetzung muss dieser nach erfolgter Oxidation in der Regel, wie zuvor beschrieben aktiviert werden.

Bei der Regenerierung kann der Katalysator mit einer löslichen Verbindung der katalytisch aktiven Komponenten in Kontakt gebracht werden. Das Inkontaktbringen kann in der Art erfolgen, dass der Katalysator mit einer wasserlöslichen Verbindung der katalytisch aktiven Komponente getränkt oder benetzt wird.

Die vorliegende Erfindung betrifft weiterhin ein Verfahren zur Herstellung von Kraftstoffadditiven, Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren, wobei in der ersten Stufe a) ein Amin erfindungsgemäß bzw. anspruchsgemäß hergestellt wird, und das so hergestellte Amin in Stufe b) bei der Herstellung von Kraftstoffadditiven, Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren eingesetzt wird.

Bei der Umsetzung von primären oder sekundären Alkoholen, Aldehyden und/oder Ketonen zu den entsprechenden Aminen ist es häufig erforderlich einen hohen Umsetzungsgrad bezüglich der eingesetzten Edukte zu erzielen, da nicht umgesetzte oder nur teilweise umgesetzte Edukte nur schwer abzutrennen sind und in den Folgeanwendungen zu unerwünschten Eigenschaften, wie Geruch und Verfärbung, führen können.
Der Vorteil der vorliegenden Erfindung besteht darin, dass das erfindungsgemäße Verfahren die Herstellung von Aminen in hoher Selektivität und Ausbeute ermöglicht. Außerdem wird die Bildung von unerwünschten Nebenprodukten verringert.

Mittels der vorliegenden Erfindung kann beim Einsatz von primären Alkoholen und/oder Aldehyden als Ausgangsstoffen die Bildung von sekundären und tertiären Aminen verringert werden bzw. die Verhältnis von primären zu sekundären Aminen eingestellt werden. Insbesondere bei der Herstellung von Aminoethoxyethanol (ADG) kann das Verhältnis von ADG zu Morpholin erhöht werden.

Mittels der vorliegenden Erfindung ist es zudem möglich den Reaktionsdruck gegenüber konventionellen Verfahren abzusenken. Eine Absenkung des Reaktionsdrucks ermöglicht in der Regel eine Verringerung der Investitionskosten, da die Anforderungen bezüglich der Reaktorsicherheit für Niederdruckverfahren nicht so hoch sind, wie für Hochdruckverfahren. Dadurch ist es möglich, die Herstellung der Amine unter milderen Reaktionsbedingungen, insbesondere bei niedrigerem Druck und/oder bei niedrigerer Temperatur durchzuführen. Somit ermöglich die vorliegende Erfindung ein wirtschaftliches Verfahren zur Herstellung von Aminen.

**Patentansprüche**

1. Verfahren zur Herstellung von Aminen durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines Katalysators, **dadurch gekennzeichnet, dass** die Querschnittsbelastung im Reaktor im Bereich von 5 kg/(m2 s) bis 50 kg/(m2 s) liegt,

2. Verfahren nach Anspruch 1, **dadurch gekennzeichnet, dass** das Festbett eine Katalysatorschüttung aus losen, geträgerten oder ungeträgerten Formkörpern ist.

3. Verfahren nach mindestens einem der Ansprüche 1 bis 2, **dadurch gekennzeichnet, dass** der Formkörper in Form von Tabletten, Ringe, Zylinder, Kugeln oder Sternstränge eingesetzt wird.

4. Verfahren nach mindestens einem der Ansprüche 1 bis 3, **dadurch gekennzeichnet, dass** die Schüttdichte der Schüttung 0,1 bis 3 kg/l beträgt.

5. Verfahren nach mindestens einem der Ansprüche 1 bis 4, **dadurch gekennzeichnet, dass** der Katalysator Pt, Pd, Co oder Ni enthält.

6. Verfahren nach mindestens einem der Ansprüche 1 bis 5, **dadurch gekennzeichnet, dass** ein Teil des Austrags (Teilaustrag) aus dem Hydrierreaktor als Rückführstrom in den Reaktor zurückgeführt (Umlaufstrom) und das Verhältnis von Umlaufstrom zum zugeführten Eduktstrom im Bereich von 0,5:1 bis 250:1 liegt.

7. Verfahren nach mindestens einem der Ansprüche 1 bis 6, **dadurch gekennzeichnet, dass** der Druck im Bereich von 15 bis 85 bar und/oder die Temperatur im Bereich von 70 bis 150°C liegt.

8. Verfahren nach mindestens einem der Ansprüche 1 bis 7, **dadurch gekennzeichnet, dass** das Verfahren in einem Schachtreaktor, Rohrreaktor oder Rohrbündelreaktor durchgeführt wird.

9. Verfahren nach Anspruch 8, **dadurch gekennzeichnet, dass** das Verhältnis von Höhe zu Durchmesser des Rohrreaktors 1:1 bis 500:1 beträgt.

10. Verfahren nach mindestens einem der Ansprüche 1 bis 9, **dadurch gekennzeichnet, dass** der Differenzdruck über die Katalysatorschüttung weniger als 1000 mbar/m beträgt.

11. Verfahren nach mindestens einem der Ansprüche 1 bis 10, **dadurch gekennzeichnet, dass** Diethylenglykol als Alkohol und Ammoniak als Aminierungsmittel eingesetzt wird.

12. Verfahren nach mindestens einem der Ansprüche 1 bis 10, **dadurch gekennzeichnet, dass** ein Polyetheralkohol als Alkohol und Ammoniak als Aminierungsmittel eingesetzt wird.

13. Verfahren zur Herstellung von Kraftstoffadditiven, Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren, **dadurch gekennzeichnet, dass** in der ersten Stufe a) ein Amin durch

ein Verfahren nach mindestens einem der Ansprüche 1 bis 12 hergestellt wird, und das so hergestellte Amin in Stufe b) bei der Herstellung von Kraftstoffadditiven, Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren eingesetzt wird.

Europäisches Patentamt
European Patent Office
Office européen des brevets

# EUROPÄISCHER RECHERCHENBERICHT

Nummer der Anmeldung
EP 13 19 6365

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | WO 2006/114427 A1 (BASF AG [DE]; EBERHARDT JAN [DE]; HOFFER BRAM WILLEM [DE]; SCHWAB EKKE) 2. November 2006 (2006-11-02)<br>* Anspruch 1; Beispiele B1,B2 *<br>----- | 1-13 | INV.<br>C07C209/16 |
| X | DE 10 2005 019373 A1 (BASF AG [DE])<br>2. November 2006 (2006-11-02)<br>* Anspruch 1; Beispiele 1,2 *<br>----- | 1-13 | |
| X | WO 2005/110969 A1 (BASF AG [DE]; HAESE FRANK [DE]; BOETTCHER ARND [MY]; STEIN BERND [DE];)<br>24. November 2005 (2005-11-24)<br>* Anspruch 1; Beispiele 1-8 *<br>----- | 1-13 | |
| X | WO 2011/042916 A1 (ALKYL AMINES CHEMICALS LTD [IN]; KATDARE SAMEER SHARAD [IN]; SOMALWAR) 14. April 2011 (2011-04-14)<br>* Anspruch 1; Beispiele 1-8 *<br>----- | 1-13 | |
| X | DE 198 59 776 A1 (BASF AG [DE] BASF SE [DE]) 29. Juni 2000 (2000-06-29)<br>* Anspruch 1; Beispiele 1-5 *<br>----- | 1-13 | RECHERCHIERTE SACHGEBIETE (IPC)<br>C07C |

Der vorliegende Recherchenbericht wurde für alle Patentansprüche erstellt

1

| Recherchenort | Abschlußdatum der Recherche | Prüfer |
|---|---|---|
| Den Haag | 14. Mai 2014 | Matés Valdivielso, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 13 19 6365

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-05-2014

| Im Recherchenbericht angeführtes Patentdokument | Datum der Veröffentlichung | Mitglied(er) der Patentfamilie | Datum der Veröffentlichung |
|---|---|---|---|
| WO 2006114427 A1 | 02-11-2006 | CN 101208319 A | 25-06-2008 |
| | | DE 102005019540 A1 | 09-11-2006 |
| | | EP 1877392 A1 | 16-01-2008 |
| | | JP 2008539200 A | 13-11-2008 |
| | | US 2008200727 A1 | 21-08-2008 |
| | | WO 2006114427 A1 | 02-11-2006 |
| DE 102005019373 A1 | 02-11-2006 | DE 102005019373 A1 | 02-11-2006 |
| | | WO 2006114417 A2 | 02-11-2006 |
| WO 2005110969 A1 | 24-11-2005 | CN 1984873 A | 20-06-2007 |
| | | DE 102004023529 A1 | 08-12-2005 |
| | | EP 1747187 A1 | 31-01-2007 |
| | | JP 2007537177 A | 20-12-2007 |
| | | US 2007232833 A1 | 04-10-2007 |
| | | WO 2005110969 A1 | 24-11-2005 |
| WO 2011042916 A1 | 14-04-2011 | CN 102574774 A | 11-07-2012 |
| | | EA 201270534 A1 | 28-09-2012 |
| | | EP 2488483 A1 | 22-08-2012 |
| | | KR 20120085777 A | 01-08-2012 |
| | | US 2012202995 A1 | 09-08-2012 |
| | | WO 2011042916 A1 | 14-04-2011 |
| DE 19859776 A1 | 29-06-2000 | DE 19859776 A1 | 29-06-2000 |
| | | US 6187957 B1 | 13-02-2001 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3275554 A
- DE 2125039 A
- DE 3611230 A
- EP 0636409 A
- EP 0742045 A
- EP 696572 A
- EP 963975 A
- EP 1106600 A2
- EP 1312600 A
- EP 1312599 A


### In der Beschreibung aufgeführte Nicht-Patentliteratur


- **ULLMANN ; KARSTEN ELLER ; ERHARD HENKES ; ROLAND ROSSBACHER ; HARTMUT HÖKE.** Aliphatic Amines in Ullmann's Encyclopedia of Industrial Chemistry. 15. Juni 2000
- **A. B. STILES.** Catalyst Manufacture - Laboratory and Commercial Preperations. Marcel Dekker, 1983
- Catalysis and Catalysts. **ULLMANN.** Ullmann's Encyclopedia Electronic Release. 2000, 28-32
- **ERTL ; KNÖZINGER ; WEITKAMP.** Handbook of Heterogenoeous Catalysis. VCH, 1997, 98 ff
- **B. STILES.** Catalyst Manufacture. Marcel Dekker, Inc, 1983, 15
- Fixed-Bed Reactors. UIlmann's Encyclopedia of Industrial Chemistry. 15. Juni 2000